# EUROPEAN PATENT APPLICATION

(11) **EP 1 742 037 A1**
(43) Date of publication of application: **10.01.2007**
(21) Application number: 06253568.7
(22) Date of filing: 07.07.2006
(51) Int. Cl.: G01N 21/55, G01N 33/58

(54) **Process to adjust the particle size in the preparation of colloidal gold**

(30) Priority: 08.07.2005 US 697746 P
(71) Applicant: DAIICHI PURE CHEMICALS CO., LTD., Chuo-ku Tokyo 103-0027 (JP)
(72) Inventor: Ito, Sachiko, Diagnostics Research Laboratories, Ryugasaki-shi, Ibaraki 301-0852 (JP); Kawamoto, Michiko, c/o Diagnostics Research Lab., Ryugasaki-shi, Ibaraki 301-0852 (JP); Kobori, Kiichiro, c/o Diagnostics Research Lab., Ryugasaki-shi, Ibaraki 301-0852 (JP); Ushizawa, Koji, Diagnostics Research Laboratories, Ryugasaki-shi, Ibaraki 301-0852 (JP)
(74) Representative: Wakerley, Helen Rachael

(57) **Abstract**

A method is provided for accurately adjusting the particle diameter of gold colloid in a simple manner, without using an expensive instrument and a skilled technique, and a gold colloid solution with a particle diameter within a desired range.
The method adjusts the particle diameter of gold colloid by estimating the particle diameter distribution width of gold colloid based on the measurement of, in addition to the maximum absorption wavelength, a ratio (Aλ_{X}/Aλₘₐₓ) of the absorbance (Aλ_{X}) at a wavelength differing from the maximum absorption wavelength to the absorbance (Aλₘₐₓ) at the maximum absorption wavelength of the gold colloid solution. The gold colloid solution obtained by the method is provided.

## Description

The present invention relates to a method for adjusting a gold colloid particle diameter. The present invention particularly relates to a method for adjusting the particle diameter of gold colloid for labeling antibodies for detection used in immunochromatography and to a gold colloid solution of which the particle diameter is adjusted.

A method of using an in-solution reducing reaction in which metal ions are reduced into colloid by adding a citrate solution as a reducing agent to a chloroauric acid solution such as a tetrachloroauric acid (III) solution and heating is frequently used for producing gold colloid solutions.

In this method, the particle diameter of gold colloid is reported to be altered by increasing or reducing the amount of the reducing agent for the amount of chloroauric acid: G.. Frens, NATURE PHYSICAL SCIENCE, 241, 20-22 (1973).

However, gold colloid with a single particle diameter cannot be obtained by only controlling the amount of the reducing agent to be added. The resulting gold colloid possesses a certain particle size distribution. Specifically, the process simultaneously produces gold colloid particles with a particle diameter outside the target range, produces gold colloid products with a particle diameter distribution significantly varying according to production batch, and does not necessarily exhibit industrially acceptable reproducibility.

For this reason, the product of each batch must be inspected to select colloid having a desired particle diameter and particle diameter distribution.

To measure particle diameter of the gold colloid produced, a method of using an electron microscope, a method of using steric-exclusion chromatography, a method of using a dynamic light scattering particle size analyzer, and the like are used. However, since all these methods require an expensive special instrument and a skilled technique for operating the instrument, the methods are not suitable for industrial routine inspection.
A certain correlation is known to exist between the particle size of gold colloid and the maximum absorption wavelength: S. L. Goodman, G.. M. Hodges, et al., J. Microscopy, 123, 201-213 (1981). However, since the particle diameter calculated based on this correlation does not necessarily correspond to the actual particle diameter, the maximum absorption wavelength cannot be used alone for measuring the particle diameter.

The present invention can provide a method for accurately measuring the particle diameter of gold colloid in a simple manner without using an expensive instrument and a skilled technique. The present invention can also provide a gold colloid solution with a particle diameter within a desired range.

As a result of extensive studies, the present inventors have found that the particle diameter distribution width of gold colloid can be estimated by measuring, in addition to the maximum absorption wavelength, a ratio (Aλ_{X}/Aλₘₐₓ) of the absorbance (Aλ_{X}) at a wavelength X differing from the maximum absorption wavelength to the absorbance (Aλₘₐₓ) at the maximum absorption wavelength of a gold colloid solution, and that the particle diameter can be adjusted based on the estimated particle diameter distribution width. This finding has led to the completion of the present invention

Accordingly, the present invention provides:
(1) A method for adjusting the particle diameter of gold colloid comprising measuring the maximum absorption wavelength (λₘₐₓ) and a ratio (Aλ_{X}/Aλₘₐₓ) of the absorbance (Aλ_{X}) at a wavelength X differing from the maximum absorption wavelength to the absorbance (Aλₘₐₓ) at the maximum absorption wavelength of a gold colloid solution.
λ ₘₐₓ: maximum absorption wavelength
Aλₘₐₓ: absorbance at the maximum absorption wavelength
Aλ_{X}: absorbance at wavelength X differing from the maximum absorption wavelength
(2) The method according to (1), wherein the wavelength differing from the maximum absorption wavelength is a wavelength of 550 to 650 nm.
(3) The method according to (1), wherein the particle diameter adjustment is adjustment of particle diameter distribution of a gold colloid solution.
(4) A method for preparing a gold colloid solution comprising measuring the maximum absorption wavelength (λₘₐₓ) and the ratio (Aλ_{X}/Aλₘₐₓ) of the absorbance (Aλ_{X}) at a wavelength X differing from the maximum absorption wavelength to the absorbance (Aλₘₐₓ) at the maximum absorption wavelength of the gold colloid solution, determining the relationship between the particle diameter, the maximum absorption wavelength (λₘₐₓ), and the ratio of absorbance (Aλ_{X}/Aλₘₐₓ), and obtaining a gold colloid solution with a desired particle diameter based on this relationship.
(5) A gold colloid solution in which the maximum absorption wavelength (λₘₐₓ) and the ratio (Aλ_{X}/Aλₘₐₓ) of the absorbance (Aλ_{X}) at a wavelength X differing from the maximum absorption wavelength to the absorbance (Aλₘₐₓ) at the maximum absorption wavelength of a gold colloid solution satisfy the following relationship:
(i) a ≤ λₘₐₓ ≤ b and
(ii) c ≤ Aλ_{X}/Aλₘₐₓ ≤ d
(6) A gold colloid solution having a desired average particle diameter in which a, b, c, and d satisfy the following relationship when the wavelength X differing from the maximum absorption wavelength is 600 nm.

| DESIRED AVERAGE PARTICLE DIAMETER | a ≤ λₘₐₓ ≤ b | c ≤ Aλ₆₀₀/Aλₘₐₓ ≤ d |
|---|---|---|
| 37 - 42 nm | 524 - 528 nm | 0.27 - 0.31 |
| 47 - 52 nm | 527 - 530 nm | 0.35 - 0.41 |
| 57 - 62 nm | 530-535 nm | 0.34 - 0.44 |
| 67 - 72 nm | 536 - 541 nm | 0.47 - 0.53 |

(7) The gold colloid solution according to (6) in which the maximum absorption wavelength (λₘₐₓ) and the ratio (Aλ_{X}/Aλₘₐₓ) of the absorbance (Aλ_{X}) at a wavelength X differing from the maximum absorption wavelength to the absorbance (Aλₘₐₓ) at the maximum absorption wavelength of a gold colloid solution satisfy the following relationship:
(i) 530 ≤ λₘₐₓ ≤ 535 and
(ii) 0.34 ≤ Aλ₆₀₀/Aλₘₐₓ ≤ 0.44
(8) The gold colloid solution according to any one of (5) to (7), which is used in immunochromatography, flow through assay, or gold colloid aggregation colorimetry.
(9) A reagent for diagnosis comprising an antibody or antigen labeled with the gold colloid solution according to (8).
(10) The reagent according to (9), wherein the antibody is an antibody for differential diagnoses of influenza.
(11) A kit for influenza differential diagnoses comprising a labeled antibody pad soaked with an antibody for differential diagnoses of influenza labeled with the gold colloid solution, in a reagent according to (10).

The particle diameter of a gold colloid solution can be accurately measured in a simple manner using the method of the present invention for adjusting a particle diameter. In addition, a gold colloid solution with a desired particle diameter can be supplied on an industrial scale.

Gold colloid herein indicates fine particles of gold dispersed in a liquid. Gold colloid has been known to exhibit a purplish red color and has conventionally been used as a coloring agent of glass, potteries, and the like, and a coloring agent of cosmetics. A gold colloid solution is used as a diagnostic reagent for immunochromatography in which immune reactions are utilized for medical use, flow through assay, gold colloid aggregation colorimetry, and the like.

A gold colloid can be produced using an in-solution reducing reaction in which metal ions are reduced into colloid by adding a citrate solution or the like as a reducing agent to a chloroauric acid solution such as a tetrachloroauric acid (III) solution and heating the mixture. The solution containing gold colloid obtained in this reaction is referred to as a gold colloid solution in the present invention.

The particle diameter of gold colloid is related to the amount of trisodium citrate added to tetrachloroauric acid (III) as mentioned above, and can be adjusted by controlling the amount to be added. Specifically, the particle diameter increases as the proportion of trisodium citrate added to tetrachloroauric acid (III) decreases. However, as mentioned above, it is difficult to adjust the particle diameter in a desired range only by controlling the amount to be added.

The maximum absorption wavelength herein refers to the maximum absorption wavelength found when the absorbance of a gold colloid solution is measured. The maximum absorption wavelength is usually in a range of 515 to 560 nm.

The wavelength differing from the maximum absorption wavelength refers to a wavelength differing from said maximum absorption wavelength of a gold colloid solution and is preferably a long wavelength, and more preferably a wavelength in a range of 550 to 650 nm.

The ratio of absorbance (Aλ_{X}/Aλₘₐₓ) in the present invention refers to a ratio of the absorbance at the wavelength differing from the maximum absorption wavelength to the absorbance at the maximum absorption wavelength of a gold colloid solution.

The present invention is based on the discovery of a positive correlation between this ratio of absorbance and the particle diameter of gold colloid.

According to the present invention, a gold colloid solution with a desired particle diameter can be obtained by previously measuring the ratio of absorbance and based on the previously determined relationship between the particle diameter, the maximum absorption wavelength (λₘₐₓ), and the ratio of absorbance (Aλ_{X}/Aλₘₐₓ).

The absorbance can be measured using any spectrophotometer having a function of measuring absorption spectrum. It is only necessary for the spectrophotometer to be able to measure an absorption spectrum at a wavelength from 400 nm to 800 nm.

In consideration of the accuracy of a spectrophotometer, the absorbance can be determined in the present invention by diluting a gold colloid solution with purified water or the like so that the absorbance at the maximum absorption wavelength may be around 1.0 and measuring the absorption spectrum of 400 nm to 800 nm using a spectrophotometer. The absorbance at the maximum absorption wavelength, absorbance at wavelength X differing from the maximum absorption wavelength, and blank absorbance at 800 nm, for example, are extracted and the ratio of the absorbance at the wavelength X to the absorbance at the maximum absorption wavelength is calculated according to the following formula.

Ratio of absorbance = (Absorbance at wavelength X - absorbance at 800 nm)/(Absorbance at the maximum absorption wavelength - absorbance at 800 nm)

According to the present invention, a gold colloid solution with a desired particle diameter can be obtained by previously measuring the ratio of absorbance and based on the previously determined relationship between the particle diameter, the maximum absorption wavelength (λₘₐₓ), and the ratio of absorbance (Aλ_{X}/Aλₘₐₓ). Specifically, when the wavelength X differing from the maximum absorption wavelength is 600 nm, for example, the gold colloid solution with the target desired average particle diameter can be selected (adjusted) when both the maximum absorption wavelength and the ratio of absorbance shown by the following Table 1 are satisfied.

**Table 1**

| DESIRED AVERAGE PARTICLE DIAMETER | a ≤ λₘₐₓ ≤ b | c ≤ Aλ₆₀₀/Aλₘₐₓ ≤ d |
|---|---|---|
| 37-42 nm | 524-528 nm | 0.27-0.31 |
| 47-52 nm | 527-530 nm | 0.35-0.41 |
| 57-62 nm | 530-535 nm | 0.34-0.44 |
| 67-72nm | 536-541 nm | 0.47-0.53 |

The gold colloid of the present invention can be used for labeling various antigens and antibodies and can be applied to any measuring methods of detecting target substances using gold colloid without any limitations. For example, the gold colloid can be used for labeling antibodies for differential diagnoses of influenza, respiratory adenovirus, or BNP.

Next, a method for preparing antibodies labeled with the gold colloid will be described. First, pH of the gold colloid solution prepared is adjusted depending on each antibody. The gold colloid solution is then mixed with an antibody solution and the mixture is stirred to bind the antibody with gold colloid. Bovine serum albumin is added as a blocking agent and the mixture is stirred further and centrifuged. The supernatant solution is removed by aspiration to obtain an antibody labeled with gold colloid as precipitate.

### Examples

### Preparation of gold colloid solution

0.61 g of chloroauric acid tetrahydrate was dissolved in 5 1 of purified water and the solution was heated. While refluxing the solution with boiling, an aqueous solution prepared by dissolving 0.48 to 0.91 g of trisodium citrate dihydrate in 10 ml of purified water was added and the mixture was stirred for ten minutes. The mixture was cooled with ice water to room temperature to obtain one batch of a gold colloid solution. The following reagents were used.

### <Reagent>

Chloroauric acid tetrahydrate: manufactured by Kanto Chemical Co., Inc.
Trisodium citrate dihydrate: manufactured by Kishida Chemical Co., Ltd.

### Evaluation of gold colloid solution

169 batches of gold colloid solutions were prepared according to the above-described method for preparing a gold colloid solution, targeting the average particle diameter of 57 to 62 nm measured by a particle size distribution analyzer. Each batch was diluted with purified water to a volume of two times to measure the average particle diameter using a particle size distribution analyzer and absorption spectrum at 400 to 800 nm using a spectrophotometer.
The distribution of the measured values for the 169 batches using the particle size distribution analyzer is shown in Table 1. Among the total 169 batches, 113 batches of gold colloid solutions with the target average particle diameter of 57 to 62 nm were obtained.

### <Measuring instrument>

Spectrophotometer: Hitachi U3310
Particle size distribution analyzer: Dynamic scattering particle size distribution analyzer NICOMP C370

### Comparative Example 1

### Establishing standard of the maximum absorption wavelength

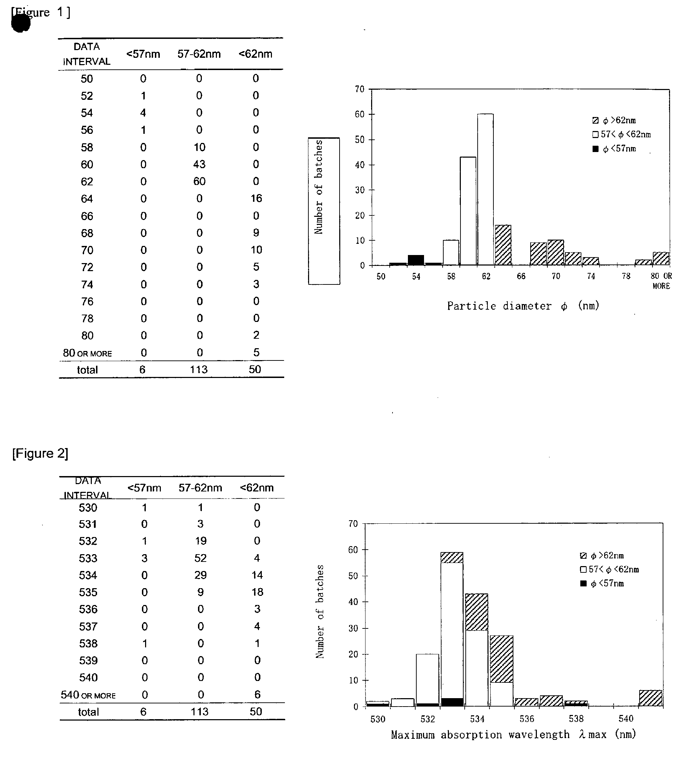

The relationship between the particle size distribution measured by the particle size distribution analyzer and the maximum absorption wavelength is shown in Figure 2. Because a certain correlation was identified between them, a standard width was given to the maximum absorption wavelength to select the target batches with the average particle diameter of 57 to 62 nm.

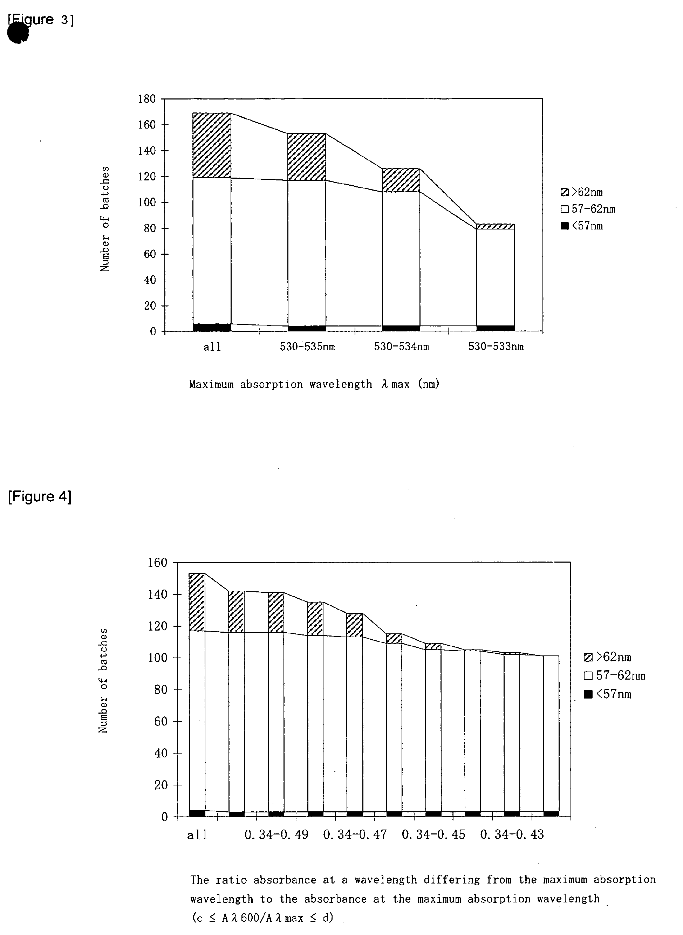

The standard of the maximum absorption wavelength of gold colloid solutions with the average particle diameter of 57 to 62 nm was set to 530 to 535 nm to confirm the particle diameters of gold colloid contained in the colloid solution. 153 batches of the gold colloid solutions satisfied the standard, confirming that 90.5% of 169 batches satisfied the standard. However, the number of batches in which the particle diameter was actually from 57 to 62 nm was 113, with 40 batches having a particle diameter outside the standard. Most of them were gold colloid solutions containing particles with a large particle diameter. Because the proportion of gold colloid with a large diameter increases as the maximum absorption wavelength increases, the test was repeated once again setting the upper limit of the maximum absorption wavelength at 534 nm. However, the number of batches in which the particle diameter was outside the standard was 22 out of 126, indicating that a significant number of batches remained outside the standard. Then, the standard of the maximum absorption wavelength was further narrowed by setting the upper limit at 533 nm. As a result, the number of batches in which the particle diameter was outside the standard was four among 83. However, about 50% of the batches were rejected by the requirement of the maximum absorption wavelength, indicating that 38 batches were rejected even though their particle diameters were within the standard. This was one third of the total 113 batches of gold colloid solutions containing particles with a diameter of 57 to 62 nm (Figure 3).

### Example 1

### Setting standard according to the maximum absorption wavelength and the ratio of absorbance

The combination of the ratio of absorbance and the maximum absorption wavelength was studied. The ratio of absorbance at 600 nm to the absorbance at the maximum absorption wavelength was calculated from the absorption spectrum. In this instance, the absorbance at 800 nm was used as a blank to determine the ratio of absorbance according to the following formula.

Ratio of absorbance = (Absorbance at 600 nm - absorbance at 800 nm)/(Absorbance at the maximum absorption wavelength - absorbance at 800 nm)

Based on the maximum and minimum values of the ratio of absorbance of gold colloid solutions having a particle diameter in the range from 57 to 62 nm, the lower limit was fixed to 0.34 and the upper limit was gradually decreased from 0.5. Gold colloid solutions meeting the conditions and the breakdown were observed (Figure 4).

As a result, in order to reduce the proportion of batches in which the particle diameter is outside the standard to 10% or less of the total batches meeting the conditions, it was required that either (i) the maximum absorption wavelength was 530 nm to 535 nm and the ratio of absorbance was 0.34 to 0.46 or (ii) the maximum absorption wavelength was 530 nm to 534 nm and the ratio of absorbance was 0.34 to 0.47. However, since gold colloid with a large particle diameter could not be excluded completely by the conditions of (i) or (ii), the upper limit of the ratio of absorbance was further decreased. As a result, almost all gold colloid particles with a large particle diameter could be excluded at a ratio of absorbance of 0.44 or more irrespective of the maximum absorption wavelength and all gold colloid solutions with an average particle diameter of 60 nm or more could be excluded at a ratio of absorbance of 0.42 or less. Thus, it was found that gold colloid solutions with a target particle diameter in the range of 57 to 62 nm can be efficiently selected, when a standard is set by combining the ratio of absorbance with the maximum absorption wavelength.

The same experiments were performed for other desired particle diameters of 37-42 nm, 47-52 nm, and 67-72 nm. The results are shown in Table 2. As a result of these experiments, it was found that gold colloid solutions with a desired average particle diameter can be obtained when the maximum absorption wavelength (λₘₐₓ) and the ratio (Aλ_{X}/Aλₘₐₓ) of the absorbance (Aλ_{X}) at a wavelength X differing from the maximum absorption wavelength to the absorbance (Aλₘₐₓ) at the maximum absorption wavelength of gold colloid solutions satisfy the relationship of Table 2.

**Table 2**

| DESIRED AVERAGE PARTICLE DIAMETER | a ≤ λₘₐₓ ≤ b | c ≤ Aλ₆₀₀/Aλₘₐₓ ≤ d |
|---|---|---|
| 37 - 42 nm | 524 - 528 nm | 0.27 - 0.31 |
| 47 - 52 nm | 527 - 530 nm | 0.35 - 0.41 |
| 57 - 62 nm | 530 - 535 nm | 0.34 - 0.44 |
| 67 - 72 nm | 536 - 541 nm | 0.47 - 0.53 |

### Example 2

### Kit for influenza differential diagnoses

### (1) Antibody

Commercially available anti-A influenza virus monoclonal antibody (mouse) and the anti-B influenza virus monoclonal antibody (mouse) were used.

### (2) Labeling with gold colloid of the present invention

The gold colloid solution with an adjusted particle diameter obtained in Example 1 was adjusted to pH 9.0 with a potassium carbonate aqueous solution. An anti-A influenza virus monoclonal antibody or anti-B influenza virus monoclonal antibody diluted with a boric acid buffer solution (pH 9.0) was added and the mixture was stirred for 10 minutes at room temperature. After the addition of an aqueous solution of bovine serum albumin and stirring for five minutes at room temperature, the mixture was centrifuged at 8,000 rpm for 45 minutes. The precipitate obtained by removing the supernatant after centrifugation by suction was suspended in a phosphate buffer solution (pH 8.0) containing bovine serum albumin, sucrose, and the like and the maximum absorption wavelength was adjusted to 7.5, thereby obtaining a gold colloid-labeled anti-A influenza virus monoclonal antibody solution or a gold colloid-labeled anti-B influenza virus monoclonal antibody solution. Equal amounts of the gold colloid-labeled anti-A influenza virus monoclonal antibody solution and gold colloid-labeled anti-B influenza virus monoclonal antibody solution were mixed to obtain a gold colloid-labeled anti-influenza antibody solution.

### (3) Labeled antibody pad

An 8.5 mm × 254 mm glass fiber pad was soaked with 128 µl of the gold colloid-labeled anti-influenza antibody solution prepared above and dried in a thermostat drier at 70°C for 30 minutes to obtain a labeled antibody pad.

### (4) Test device

The gold colloid-labeled anti-A influenza virus monoclonal antibody solution and gold colloid-labeled anti-B influenza virus monoclonal antibody solution were linearly applied to a nitrocellulose membrane and dried in a thermostat drier at 70°C for 30 minutes to obtain an antibody-immobilized membrane. The antibody-immobilized membrane, the labeled antibody pad, and an absorption paper were stuck on an adhesive sheet made from plastic, which was cut into a width of 7.1 mm to obtain test strips. The test strips were put into a plastic container equipped with an opening for adding test sample solutions and another opening in which lines of an anti-A influenza virus monoclonal antibody and an anti-B influenza virus monoclonal antibody were exposed thereby obtaining a test device.

### (5) Kit

The kit for influenza differential diagnoses comprises a plastic tube containing a fluid for test sample dilution, which is a phosphate buffer solution (pH 7.6) containing bovine serum albumin, and the test device prepared above.

### (6) Diagnostic test

A test sample was colleted from a patient suspected of influenza virus infection using a swab. The swab was inserted into the plastic tube containing the fluid for test sample dilution, the tube was moved up and down seven or eight times to suspend the sample, and the swab was removed from the tube. A filter nozzle was attached to the opening of the tube and five drops of the test sample solution were dropped into the opening for adding sample solutions of the test device. After 15 minutes, lines of a purplish red color appearing in the judgment window of the testing device were inspected to confirm that the differential diagnosis of influenza was possible.

Thus, a simple method for adjusting the particle diameter of a gold colloid solution prepared can be provided according to the present invention. Specifically, a gold colloid solution with a target particle diameter can be easily selected at a high rate by combining the maximum absorption wavelength with a ratio of the absorbance at a wavelength differing from the maximum absorption wavelength to the absorbance at the maximum absorption wavelength, measured using a common spectrophotometer.

### Brief Description of the Drawings

Figure 1 shows a histogram according to particle diameters.
Figure 2 shows a histogram according to maximum absorption wavelengths.
Figure 3 shows the result of standard determination according to maximum absorption wavelengths.
Figure 4 shows the result of standard determination according to combination of maximum absorption wavelengths and ratios of absorbance.

## Claims

1. A method for adjusting the particle diameter of gold colloid comprising measuring the maximum absorption wavelength (λₘₐₓ) and a ratio (Aλₓ/Aλₘₐₓ) of the absorbance (Aλₓ) at a wavelength X differing from the maximum absorption wavelength to the absorbance (Aλₘₐₓ) at the maximum absorption wavelength of a gold colloid solution.
λ ₘₐₓ: maximum absorption wavelength
Aλₘₐₓ: absorbance at the maximum absorption wavelength
Aλₓ: absorbance at wavelength X differing from the maximum absorption wavelength

2. The method according to claim 1, wherein the wavelength differing from the maximum absorption wavelength of 550 to 650 nm.

3. The method according to claim 1, wherein the particle diameter adjustment of particle diameter distribution of a gold colloid solution.

4. A method for preparing a gold colloid solution comprising measuring the maximum absorption wavelength (λₘₐₓ) and the ratio (Aλₓ/Aλₘₐₓ) of the absorbance (Aλₓ) at wavelength X differing from the maximum absorption wavelength to the absorption (Aλₘₐₓ) at the maximum absorption wavelength of the gold colloid solution, determining the relationship among the particle diameter, the maximum absorption wavelength (λₘₐₓ), and the ratio of absorbance (Aλₓ/Aλₘₐₓ), and obtaining a gold colloid solution with a desired particle diameter based on this relationship.

5. A gold colloid solution in which the maximum absorption wavelength (λₘₐₓ) and the ratio (Aλₓ/Aλₘₐₓ) of the absorbance (Aλₓ) at a wavelength X differing from the maximum absorption wavelength to the absorbance (Aλₘₐₓ) at the maximum absorption wavelength of gold colloid solution satisfy the following relationship:
(i) a ≤ λₘₐₓ ≤ b and
(ii) c ≤ Aλₓ ≤ Aλₘₐₓ ≤ d

6. A gold colloid solution having a desired average particle diameter in which a, b, c and d satisfy the following relationship when the wavelength X differing from the maximum absorption wavelength is 600nm:
| DESIRED AVERAGE PARTICLE DIAMETER | a ≤ λₘₐₓ ≤ b | c ≤ Aλ₆₀₀/Aλₘₐₓ ≤ d |
|---|---|---|
| 37-42 nm | 524-528 nm | 0.27-0.31 |
| 47-52 nm | 527-530 nm | 0.35-0.41 |
| 57-62nm | 530-535nm | 0.34-0.44 |
| 67-72nm | 536-541 nm | 0.47-0.53 |

7. The gold colloid solution according to claim 6 in which the maximum absorption wavelength (λₘₐₓ) and the ratio (Aλ_{X}/Aλₘₐₓ) of the absorbance (Aλ_{X}) at a wavelength X differing from the maximum absorption wavelength to the absorbance (Aλₘₐₓ) at the maximum absorption wavelength of a gold colloid solution satisfy the following relationship:
(i) 530 ≤ λₘₐₓ ≤ 535 and
(ii) 0.34 ≤ Aλ₆₀₀/Aλₘₐₓ ≤ 0.44

8. The gold colloid solution according to any one of claim 5 to 7, which is used in immunochromatography, flow through assay, or gold colloid aggregation colorimetry.

9. A reagent for diagnosis comprising an antibody or antigen labeled with the gold colloid solution according to claim 8.

10. A reagent according to claim 9, wherein the antibody is an antibody for differential diagnoses of influenza.

11. A kit for influenza differential diagnoses comprising a labeled antibody pad soaked with an antibody for differential diagnoses of influenza labeled with the gold colloid solution in a reagent according to claim 10.
